# EUROPÄISCHE PATENTSCHRIFT

(11) **EP 2 578 263 B1**
(45) Veröffentlichungstag und Bekanntmachung des Hinweises auf die Patenterteilung: **08.01.2014**
(21) Anmeldenummer: 12183870.0
(22) Anmeldetag: 11.09.2012
(51) Int. Cl.: A61M 25/00

(54) **Medizinische Vorrichtung und Führungseinrichtung hierfür**
Medical device and guide device for same
Dispositif médical et dispositif de guidage à cet effet

(30) Priorität: 04.10.2011 US 201161542824 P
(43) Veröffentlichungstag der Anmeldung: 10.04.2013
(73) Patentinhaber: BIOTRONIK SE & Co. KG, 12359 Berlin (DE)
(72) Erfinder: Widdecke, Heinrich, 14547 Beelitz (DE)
(74) Vertreter: Lindner-Vogt, Karin L.

(56) Entgegenhaltungen:
- WO-A2-2007/000148
- US-A1- 2004 186 377
- US-A1- 2004 230 271

## Beschreibung

Die vorliegende Patentanmeldung betrifft eine Führungseinrichtung mit einem Polymer- und/oder Kunststoffschlauch, insbesondere Schlauch enthaltend ein Polysiloxan, in dessen durchgehender Öffnung ein in der Öffnung bewegliches Seil oder Band geführt ist. Die Patentanmeldung bezieht sich ferner auf eine medizinische Vorrichtung, insbesondere auf einen Katheter, mit einer solchen Führungseinrichtung sowie ein Verfahren zur Herstellung einer derartigen Führungseinrichtung bzw. einer derartigen medizinischen Vorrichtung. Eine solche Vorrichtung ist aus der US 20040186377 bekannt.

Als medizinische Vorrichtung im Sinne der Patentanmeldung werden längliche, vorübergehend oder dauerhaft in einen lebenden Körper einführbare Katheter oder Elektrodenleitungen umfasst. Elektrodenleitungen sind permanente Implantate zum Verbleib in Herz, Gehirn oder Rückenmark, welche dazu dienen, therapeutische oder diagnostische Signale zwischen einem elektromedizinischen Implantat (implantierbare Herzschrittmacher, Defibrillatoren, Neurostimulatoren oder Cochlearimplantate) und einer Elektrodenfläche am entgegen gesetzten Ende der Elektrodenleitung auszutauschen. Als Katheter wird ein Röhrchen oder Schlauch bezeichnet, das/der in einen zu behandelnden Körperhohlraum eingeführt werden kann. Solche Schläuche werden in verschiedenen Durchmessern angeboten. Die Körperhohlräume wie Harnblase, Magen, Darm, Gefäße usw., aber auch Ohr und Herz, werden mit einem Katheter sondiert, entleert, gefüllt oder gespült. Dies geschieht aus diagnostischen oder therapeutischen Gründen.

Alle im Sinne der Patentanmeldung als medizinische Einrichtungen bezeichnete sind in der Regel gleich aufgebaut. Sie bestehen aus einem proximalen Ende und einem in Richtung der Behandlungsstelle weisendes distales Ende. An diesem Ende sind therapie- oder diagnoserelevante Einrichtungen angebracht wie beispielsweise Elektroden zur Stimulation von Körperorganen oder zur Abfühlung von Körpersignalen. Das proximale Ende dient zum einen als Schnittstelle zu einem Implantat oder zu externen Steuer-, Therapie- oder Diagnosegeräten und kann zusätzlich dazu dienen, eine Bedienung durch einen Arzt zu ermöglichen, beispielsweise zur Steuerung des flexiblen distalen Endes mittels einer Steuereinrichtung. Zwischen dem proximalen und distalen Ende der medizinischen Einrichtung befindet sich ein lang gestreckter Körper mit mindestens einem Schlauch oder Röhrchen. Ein solcher Schlauch besteht aus einer durchgehenden Öffnung - auch Lumen bezeichnet, die sich entlang der Längsachse des Schlauches erstreckt. Besteht der Körper aus mehreren parallel verlaufenden Schläuchen, die eine gemeinsame Einheit bilden, besteht er aus mehreren Lumen und wird daher als Multilumenschlauch bezeichnet. Jedes der Lumen, insbesondere die in radialer Richtung von der Längsachse versetzten Außenlumen, kann dabei die durchgehende Öffnung des Schlauches einer Führungseinrichtung bilden. Führungseinrichtungen dienen dazu, Seile oder Bänder zu führen. Die Seile oder Bänder in den Führungseinrichtungen können dabei insbesondere der Aufgabe dienen, die Steuerbarkeit des distalen Endes zu ermöglichen und/oder therapeutische oder diagnostische Signale zu übertragen.
Die unter dem Begriff medizinische Vorrichtungen umfassten Katheter, insbesondere so genannte Ablationskatheter, die vor allem in der Elektrophysiologie zur thermischen Verödung eines Reizleitungspfades im Herz genutzt werden, weisen einen soeben genannten Multilumenschlauch als Katheterkörper auf, bei dem mehrere oder alle Außenlumen als Lumen einer Führungseinrichtung dienen können. Das heißt, dass in einem oder jedem der Lumen ein Seil - in diesem Fall ein Steuerseil oder Steuerband - geführt ist. Die Steuerseile verlaufen in je einem der Lumina. Mit definierten Zugbewegungen an den Seilen lässt sich die Katheterspitze aus der Längsachse heraus lenken und so die Katheterspitze an die gewünschte zu therapierende Geweberegion führen. Zur Steuerung werden die Steuerseile oder -bänder mittels eines Handgriffes vom Arzt bedient, was zu einer Bewegung des Seils relativ zum Lumen führt. An der Katheterspitze befinden sich diverse Elektrodenflächen, mit deren Hilfe Radiofrequenzsignale in die Geweberegion zur Ablation geleitet werden.

Eine Relativbewegung zwischen einem Seil und Lumen geschieht auch bei permanent im Körper verbleibenden Elektrodenleitungen, welche ebenfalls im Sinne der Patentanmeldung unter dem Begriff Katheter zusammen gefasst sind.

Deren Körper - Elektrodenkörper genannt - ist im Prinzip so aufgebaut wie der soeben beschriebene Katheterkörper und ist mit diesem gleichgesetzt. Im Unterschied dazu verbleibt aber die Elektrodenleitung permanent im Körper und ist deshalb anderen Belastungen ausgesetzt. Ebenso umfasst eine solche Elektrodenleitung in den seltensten Fällen Steuerungsseile, sondern umfasst zur Überleitung der elektrischen Signale einerseits vom elektromedizinischen Implantat - am proximalen Ende der Elektrodenleitung - zu einer Elektrode am distalen Ende der Elektrodenleitung oder umgekehrt elektrisch leitfähige Zuleitungsseile oder -bänder. Diese elektrischen Zuleitungsseile verlaufen ebenfalls im Lumen eines Schlauches. Der Elektrodenkörper kann ebenfalls aus mehreren Schläuchen in einem Verbund hergestellt werden, wobei in einem oder mehreren der Schlauchlumen die Leitungsseile verlaufen.

Durch das permanent in Bewegung befindliche Herz wird die Elektrodenleitung dauernder Biegebelastung ausgesetzt, was ebenfalls zu einer Relativbewegung zwischen jedem Schlauch in einem Elektrodenkörper und Leitungsseil führt.

Im Folgenden wird das System aus Schlauch (Röhrchen) mit einer durchgehenden Öffnung (Lumen) und darin verlaufendem Seil (Steuerseil oder elektrisches Zuleitungsseil einer Elektrodenleitung) als Führungseinrichtung bezeichnet. Das bedeutet, dass in einem Körper einer medizinischen Vorrichtung als Verbund mehrerer Schläuche jeder Schlauch, in dem ein Seil geführt ist, eine Führungseinrichtung ist. Die Führungseinrichtung bildet das Herzstück der medizinischen Vorrichtung. Für die verschiedenen Einsatzmöglichkeiten kann diese medizinische Vorrichtung noch weitere Einrichtungen, beispielsweise einen Ballon, Anschlüsse für Messgeräte, eine Spritze für die Bereitstellung einer Flüssigkeit zur Behandlung (Medikament oder andere pharmazeutisch aktive Substanz) oder Diagnose (z.B. mit Kontrastmittel) oder dergleichen umfassen. Zudem kann ein weiteres Lumen existieren, in dem temporär ein Führungsdraht aufgenommen ist.

Die Führungseinrichtung nach dem Gegenstand der vorliegenden Patentanmeldung kann auch in anderen als den genannten medizinischen Vorrichtungen, bspw. in minimalinvasiven Instrumenten verwendet werden. In diesen Vorrichtungen erfolgt die Steuerung ebenfalls mittels Führungs- oder Zugseil(en), welche(s) sich relativ zu einem Schlauch oder Röhrchen bewegen.

In einer derartigen Führungseinrichtung, in der eine Relativbewegung zwischen zwei verschiedenen Materialien stattfindet (meist Polymer und/oder Kunststoffmaterial auf der Seite des Schlauchs und metallisches Material auf der Seite des Seils), wird ein hoher Verschleiß beobachtet. Es kann dabei vorkommen, dass sich ein Seil durch das umgebende Schlauchmaterial hindurch reibt. Bei der Steuerung von Kathetern hingegen wird eine erhöhte Reibkraft festgestellt, die dazu führt, dass die Steuerung schwerläufig ist und so die Therapie erschwert wird oder sich in die Länge zieht.

Die Aufgabe der vorliegenden Patentanmeldung besteht somit darin, eine Führungseinrichtung bzw. eine medizinische Vorrichtung mit jeweils wesentlich verringertem Verschleiß oder Reibung zu schaffen. Die Aufgabe besteht ferner darin, ein einfaches und kostengünstiges Verfahren zur Herstellung einer solchen Führungseinrichtung bzw. einer derartigen medizinischen Vorrichtung anzugeben.

Die obige Aufgabe wird durch eine Führungseinrichtung gelöst, welche auf der äußeren Oberfläche des Seils und/oder der inneren Oberfläche der durchgehenden Öffnung einen Belag aufweist, der überwiegend aus im Wesentlichen kugel- oder zylinderförmigen und zumindest auf der jeweiligen Oberfläche frei beweglichen Partikeln besteht.

Gemäß des Gegenstandes der Patentanmeldung werden somit feinkörnige Partikel zwischen Schlauch und Seil angeordnet, die aufgrund ihrer Kugel- oder Zylinderform und ihrer freien Beweglichkeit auf der jeweiligen Oberfläche einen rollenden Belag bzw. eine rollfähige Schicht ergeben. Es wird also die nach dem Stand der Technik vorliegende Gleitreibung zwischen den Materialien, die zu dem hohen Verschleiß oder Schwergängigkeit geführt hat, vermieden und durch Rollreibung ersetzt. Der Verschleiß und die Schwergängigkeit werden hierdurch ganz erheblich reduziert und damit die Nutzungsdauer der die Führungseinrichtung aufweisenden medizinischen Vorrichtung erheblich verlängert bzw. deren Nutzung vereinfacht. Ebenso wird die Flexibilität erheblich vergrößert.

Das Wesentliche an dem genannten Belag besteht somit darin, dass die auf der Oberfläche des Seils bzw. des Schlauchs beweglichen Partikel die Relativbewegung zwischen Seil und Schlauch begünstigen und Haftreibung verhindern. Dabei sind die Partikel nicht homogen, sondern meist unregelmäßig auf der Oberfläche des Seils bzw. der Oberfläche des Schlauchs verteilt und ergeben auch keine, die jeweilige Oberfläche vollständig bedeckende Schicht, da die Möglichkeit der freien Bewegung der Partikel auf der jeweiligen Oberfläche gegeben sein soll. Vorzugsweise sind die Partikel nicht nur auf der jeweiligen Oberfläche sondern im dreidimensionalen Raum frei beweglich. Sie können dann sowohl auf der Seiloberfläche als auch auf der inneren Oberfläche des Schlauchs rollen.

Im Rahmen der vorliegenden Patentanmeldung bedeutet der Ausdruck "im Wesentlichen kugel- oder zylinderförmig" hinsichtlich der Partikel, dass diese in etwa eine Kugel- oder Zylinderform aufweisen. Sie müssen nicht als ideale Zylinder oder Kugeln geformt sein, Abweichungen von dieser Form sind möglich und durch die vorliegende Patentanmeldung umfasst. Die Form der Partikel muss sicherstellen, dass die Partikel auf der Oberfläche des Seils bzw. der inneren Oberfläche des Schlauchs bei einer Relativbewegung zwischen Seil und Schlauch rollen können. Deshalb ist eine geringfügige Abweichung von der idealen Kugel- bzw. Zylinderform ist für die Eigenschaft des Rollens unerheblich.

In einem Ausführungsbeispiel der vorliegenden Patentanmeldung sind die Partikel des Belags nicht zerreibbar und/oder nicht degradierbar und können zusätzlich biokompatibel sein. Diese Eigenschaften der Partikel sind insbesondere bei einem sehr langen Einsatz der Führungseinrichtung im Körper wünschenswert, so dass die Roll-Eigenschaft der Partikel nicht im Lauf der Einsatzzeit verschlechtert und damit der Verschleiß erhöht wird.

Bevorzugt sind die Partikel des Belages aus einem der folgenden Materialien hergestellt: Korund, Quarzsand, Polyamid, Polyimid oder Teflon. Es ist auch möglich, verschiedene Partikel aus einem oder mehreren der genannten Materialien zu mischen.

Es ist ferner bevorzugt, wenn die Partikel einen Durchmessermittelwert größer oder gleich 20 µm aufweisen. Weiter bevorzugt ist dieser Durchmessermittelwert größer gleich 20 µm und nicht größer als 60 um, besonders bevorzugt weisen die Partikel einen Durchmessermittelwert von 50 µm auf. Hierbei wird zur Mittelwertbestimmung des Durchmessers eine mikroskopische Aufnahme der Partikel hergestellt und in dieser der Durchmesser von mindestens 10 Partikeln ausgemessen, deren arithmetischer Mittelwert dann den mittleren Durchmesser der Partikel ergibt. Die genannten Abmessungen der Partikel sind von Vorteil, da bei der Verwendung von feinerem Material kein Rolleffekt mehr möglich ist und größere Partikel keine Bewegungsfreiheit mehr hätten.

In einem bevorzugten Ausführungsbeispiel besteht der Schlauch der Führungseinrichtung aus einem Mittellumen und mindestens einem Außenlumen, wobei bevorzugt eines oder mehrere der Außenlumen das Seil oder die Seile aufnehmen. Das Material des Schlauches ist bevorzugt Silikonmaterial. Der Mehrlumenschlauch kann auch noch weitere außen liegende Schichten besitzen, wobei die Schichtstruktur des Schlauchs dazu dient, für die Anwendung günstige Eigenschaften zu erzielen, beispielsweise gute Gleiteigenschaften bzw. Sterilisierbarkeit durch eine entsprechende außen liegende Schicht und günstige mechanische Eigenschaften (Elastizität, einen kleinen Knickradius, gute Biegeeigenschaften etc.).

Die obige Aufgabe wird ferner durch eine medizinische Vorrichtung, insbesondere einen Katheter oder eine implantierbare Elektrodenleitung gelöst, welche(r) eine Führungseinrichtung mit dem oben beschriebenen Belag aufweist.

Die medizinische Vorrichtung hat die oben im Zusammenhang mit der Führungseinrichtung gemäß Gegenstand der Patentanmeldung angegebenen Vorteile.

Eine weitere Lösung zur obigen Aufgabenstellung stellt das einfache und kostengünstig durchführbare Verfahren zur Herstellung einer Führungseinrichtung dar, bei dem nach Bereitstellung des Kunststoff- und/oder Polymerschlauchs ein Dosierkolben, der beispielsweise aus einem Drahtgeflecht besteht, zuerst durch eine Anhäufung von Partikeln, welche im Wesentlichen kugel- oder zylinderförmig und voneinander unabhängig beweglich sind, und anschließend durch die durchgehende Öffnung des Kunststoff- und/oder Polymerschlauchs bewegt wird.

Hierbei wird der Dosierkolben vorzugsweise mittels eines Zugseils durch die Öffnung in dem Kunststoff- und/oder Polymerschlauch hindurch gezogen und die kugel- oder zylinderförmigen Partikel, die beim Hindurchziehen durch die Anhäufung der Partikel aufgenommen wurden, werden auf die Innenseite des Schlauchs übertragen.

Weiter von Vorteil ist, wenn der Dosierkolben nach dem ersten Einbringen der Partikel in die durchgehende Öffnung des Schlauchs noch mindestens ein weiteres Mal durch die durchgehende Öffnung bewegt wird, um die Partikel in der Öffnung besser zu verteilen.

Nach dem Aufbringen der kugel- oder zylinderförmigen Partikel auf die innere Oberfläche der durchgehenden Öffnung des Schlauchs wird das Seil in die Öffnung eingeführt und hierdurch die Führungseinrichtung fertig gestellt.

Die obige Aufgabe wird außerdem durch ein Verfahren zur Herstellung einer medizinischen Vorrichtung, insbesondere eines Katheters, gelöst, bei dem zunächst die Führungseinrichtung nach dem oben beschriebenen Verfahren hergestellt und anschließend die weiteren Elemente der medizinischen Vorrichtung an der Führungseinrichtung angeordnet werden, beispielsweise mit dieser verbunden werden. Als weiterer Schritt nach der Herstellung der Führungseinrichtungen können vorzugsweise mehrere dieser Führungseinrichtungen nach ihrer Herstellung zu einem Verbund zusammengeführt werden, um so ein Multilumenschlauch zu formen. Weiter bevorzugt können zusätzlich zu den Führungsdrähten weitere Schläuche zu dem Verbund hinzugefügt werden, damit beispielsweise eine durchgehende Öffnung für einen temporär vorhandenen Führungsdraht vorhanden ist.

Weitere Merkmale, Vorteile und Anwendungsmöglichkeiten ergeben sich auch aus der nachfolgenden Beschreibung von Ausführungsbeispielen und der Figuren. Dabei bilden alle beschriebenen und/oder bildlich dargestellten Merkmale für sich oder in beliebiger Kombination den Gegenstand der vorliegenden Patentanmeldung, auch unabhängig von ihrer Zusammenfassung in den Ansprüchen oder deren Rückbezügen.

Es zeigen schematisch:
- Fig. 1: einen Längsschnitt durch einen Abschnitt einer Führungseinrichtung gemäß des Gegenstandes der Patentanmeldung,
- Fig. 2: eine Ansicht eines Dosierkolbens mit Zugseil von der Seite,
- Fig. 3: einen Schlauch einer Führungseinrichtung mit Dosierkolben und Führungsseil in einer perspektivischen Ansicht von der Seite,
- Fig. 4: den Schlauch der Führungseinrichtung gemäß Fig. 3 beim Einbringen der in einem Behälter angeordneten Partikel in einer perspektivischen Ansicht von der Seite,
- Fig. 5: einen Querschnitt durch eine Führungseinrichtung nach einem Verschleißtest an einem ersten Vergleichsbeispiel,
- Fig. 6: die verschlissene Führungseinrichtung des ersten Vergleichsbeispiels in einer Ansicht von der Seite,
- Fig. 7: einen Querschnitt durch eine Führungseinrichtung nach einem Verschleißtest an einem zweiten Vergleichsbeispiel und
- Fig. 8: einen Querschnitt durch eine Führungseinrichtung gemäß des Gegenstandes der Patentanmeldung nach einem Verschleißtest.

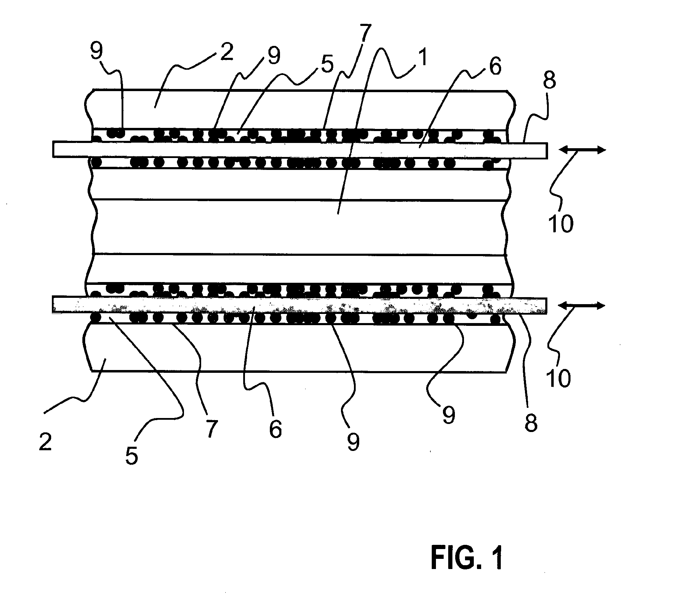

Fig. 1 zeigt den Längsschnitt einer medizinischen Vorrichtung 100 mit zwei Führungseinrichtungen des Gegenstandes der Patentanmeldung. Die Vorrichtung besteht aus einem Mehrlumenschlauch mit einem Innenschlauch 1 und mindestens einem sich in radialer Richtung zum Mittellumen versetzten Außenschlauch 2, welcher je ein Außenlumen 5 als durchgehende Öffnung aufweist. Der Innenschlauch 1 und der mindestens eine Außenschlauch 2 sind dabei zu einem Verbund (Multilumenschlauch) zusammengefügt. Der Multilumenschlauch - also auch die Schläuche 1 und 2 - besteht aus einem Polymer- und/oder Kunststoffschlauch, welcher vorzugsweise Polysiloxan enthält.

In den Außenlumen 5 sind die Seile 6 angeordnet. Das Seil 6 ist aus 7x7 verseilten Einzeldrähten mit einer teflonartigen Umhüllung hergestellt. Der in Fig. 1 gezeigte Längsschnitt stellt lediglich zwei dieser Außenlumen 5 dar.

Es versteht sich, dass die Vorrichtung alternativ auch lediglich eine durchgehende Öffnung mit einem Seil oder jede andere Anzahl von durchgehenden Öffnungen aufweisen kann. Alternativ kann eine durchgehende Öffnung sich auch in dem Innenschlauch erstrecken.

Zwischen der inneren Oberfläche 7 des Außenschlauchs 2 welche die durchgehende Öffnung 5 ausbildet, und der äußeren Oberfläche 8 des Seils 6 sind eine Reihe von im Wesentlichen kugelförmigen Partikeln 9 bestehend aus Korund, Quarzsand, Polyamid, Polyimid oder Teflon mit einem mittleren Durchmesser von größer oder gleich 20 µm, bevorzugt nicht größer als 60 µm, besonders bevorzugt mit einem mittleren Durchmesser von 50 µm angeordnet.

Die Partikel 9 bewirken, dass bei einer Relativbewegung zwischen Seil 6 und Außenschlauch 2 in Längsrichtung (veranschaulicht durch Doppelpfeil 10) anstelle der sonst vorliegenden Haftreibung zwischen der inneren Oberfläche 7 des Außenschlauchs 2 und der äußeren Oberfläche 8 des Seils 6 Rollreibung stattfindet, die zu einem wesentlich geringeren Verschleiß führt. Die Rollreibung erfolgt zwischen der inneren Oberfläche 7 und den Partikeln 9 bzw. den Partikeln 9 und der Oberfläche 8 des Seils 6. Das Seil 6 reibt sich aufgrund dessen nicht mehr in das Silikonmaterial hinein oder durch diesen hindurch.

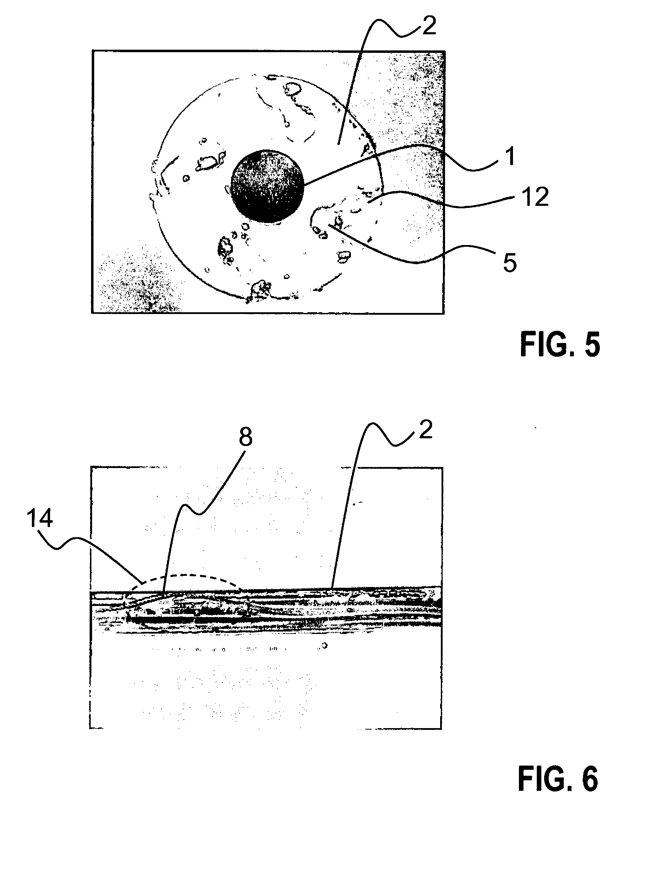

In einer in Fig. 1 gezeigten Konfiguration wurde an einem Prüfling, der den Aufbau gemäß des Gegenstandes der Patentanmeldung aufweist, ein spezieller Verschleißtest , durchgeführt und mit den Ergebnissen des gleichen Verschleißtests zu zwei Prüflingen verglichen, die nicht die erfindungsgemäße Lösung verwirklichten. Die Prüflinge hatten eine Länge von ca. 60 mm. Die Ergebnisse sind in den Figuren 5 bis 8 dargestellt. Der Verschleißtest besteht darin, dass bei der Erzeugung einer Drehbewegung des Prüflings in einer bestimmten Art eine relative Bewegung zwischen den Seilen und dem Außenlumen erzeugt wird. So soll die Bewegung einer im schlagenden Herz dauerimplantierten medizinischen Vorrichtung simuliert werden.

In einem ersten Test hatte der Prüfling einen Mehrlumenschlauch, in dessen vier Außenlumen 5 jeweils ein Seil 8 aus 7x7 verseilten Einzeldrähten mit einer teflonartigen Umhüllung angeordnet war, über 2,5 Millionen Zyklen bei 7.500 Umdrehungen pro Minute bewegt. Weder der Außenlumen 5 noch das Seil enthielten auf ihrer Oberfläche den Belag (Rollschicht) gemäß des Gegenstandes der Patentanmeldung. Nach Ende der Belastung wurde der Verschleiß in dem Schlauch 2 beobachtet. Hierbei ergab sich, dass sich ein Seil 8 durch den Außenschlauch 2 hindurch gerieben hat (siehe Bereich 14 in Fig. 6) und auch die anderen drei Seile 8 sich deutlich in das Silikonmaterial eingerieben haben. Die entsprechend entstandenen Erweiterungen 12 der durchgehenden Öffnungen 5 sind in dem Querschnitt der Fig. 5 deutlich erkennbar.

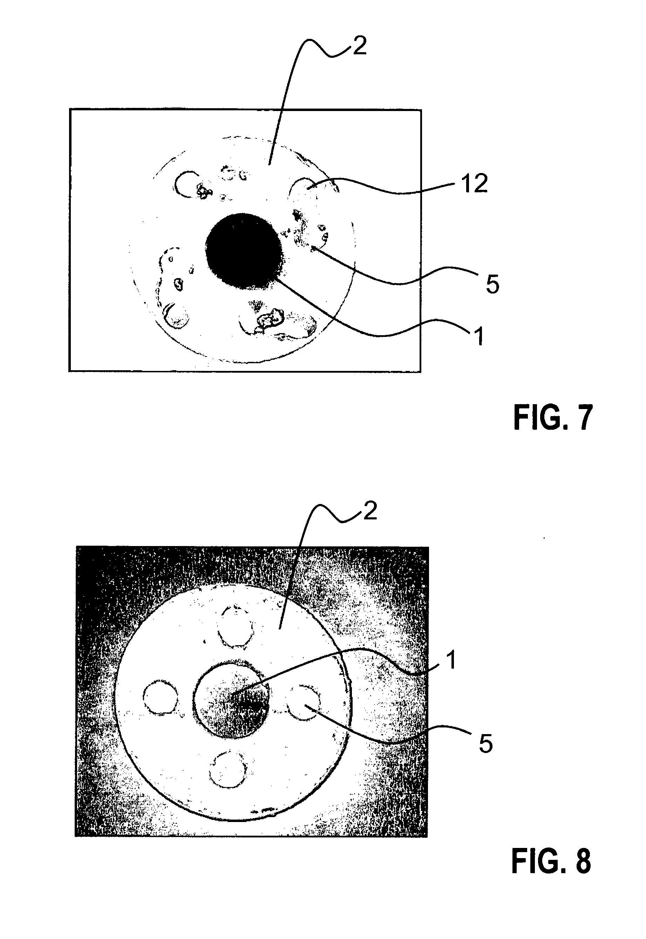

Auch bei einer sonst gleichen Konfiguration wie in dem ersten Test und einer zusätzlich auf die der inneren Oberfläche des Schlauchs aufgebrachten Beschichtung bestehend aus hochfestem Zweikomponenten-Silikonmaterial mit der Bezeichnung MED-6670 der Firma NuSil wurde nach der gleichen Belastung in einem zweiten Test ein deutlicher Verschleiß festgestellt. In Fig. 7 ist ein Querschnitt eines derartigen Prüflings nach Ende der Belastung dargestellt. Auch hier haben sich die Seile 6 fast durch die Schlauchwand hindurch gerieben. Deutlich sind die durch den Verschleiß entstandenen Erweiterungen 12 der Öffnungen 5 erkennbar.

In einem dritten Test wurde dann eine Führungseinrichtung gemäß des Gegenstandes der Patentanmeldung in gleicher Konfiguration wie in dem ersten Test mit vier durchgehenden Öffnungen und darin angeordneten Seilen, zwischen denen ein Belag aus im Wesentlichen kugelförmigen Partikel bestehend aus Polyamid-Partikeln vorgesehen ist, durchgeführt. Nach einer Laufzeit von 100 Millionen Zyklen bei 7.500 Umdrehungen pro Minute, d.h. bei einer 40-fach längeren Belastung, konnte anhand des in Fig. 8 gezeigten Querschnitts, in dem die durchgehenden Öffnungen 5 geringfügige Abweichungen von der ideal runden Form zeigen, nur ein geringfügiger Verschleiß festgestellt werden.

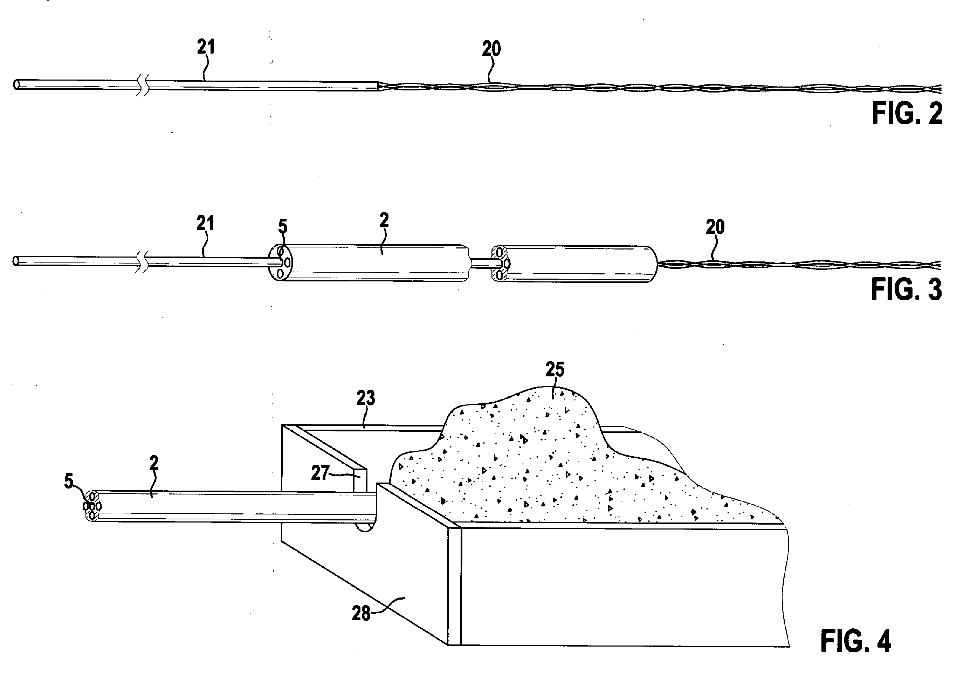

Nun soll die Herstellung einer Führungseinrichtung gemäß des Gegenstandes der Patentanmeldung anhand der Figuren 2 bis 4 beschrieben werden. Beispielhaft wird die Herstellung anhand eines einzelnen Schlauches beschrieben. Prinzipiell ist dieses Herstellungsverfahren auch für medizinische Vorrichtungen wie Katheter- oder Elektrodenkörper geeignet, die einen Mehrlumenschlauch mit Führungseinrichtungen wie in Fig. 1 genannt umfassen.

Fig. 2 zeigt einen Dosierkolben 20, der mit einem Zugseil 21 verbunden ist. Hierbei ist der Dosierkolben 20 ein Kolben, der die Partikel in trockener Form oder zusammen mit einer Flüssigkeit aufnehmen und über eine längere Strecke innerhalb der Öffnung 5 des Schlauchs 2 der Führungseinrichtung verteilen kann. Der Dosierkolben 20 besteht bspw. aus einer Drahtwendel mit mindestens zwei lang gestreckten, gewundenen Drähten oder einem Geflecht aus derartigen Drähten. Die zu befördernde Menge an Partikeln wird unter Anderem durch die Länge des Dosierkolbens 20 reguliert. Für längere Schläuche ist die Häufigkeit des unten beschriebenen Vorgang des Durchziehens je nach Bedarf zu wiederholen. Das Zugseil 21 ist länger als die durchgehende Öffnung 5 des mit Partikel zu versehenen Außenlumen 5.

Der Dosierkolben 20 ist mit dem Zugseil 21 verbunden, welches beim Hindurchführen des Dosierkolbens 20 durch die Öffnung 5 des Schlauchs 2 der Führungseinrichtung als Kraftüberträger und Handhabungsmittel dient.

Zunächst wird das Zugseil 21, wie in Fig. 3 dargestellt, durch die mit Partikel zu versehene durchgehende Öffnung 5 gezogen. Das Zugseil 21 wird so weit durch die Öffnung 5 hindurch gezogen, bis der Dosierkolben 20 an einem Ende des Außenlumen 5 anliegt. Bei dem in Fig. 3 gezeigten Beispiel ist dies das rechte Ende des Silikonschlauches.

In dieser Stellung wird der Silikonschlauch in den Schlitz 27 einer vorderen Wand 28 eines Behälters (Vorratsbox) 23 mit einer Anhäufung 25 von den in die durchgehende Öffnung einzubringenden kugel- oder zylinderförmigen Partikeln eingesteckt. Hierbei wird der Silikonschlauch durch den Schlitz 27 festgeklemmt. Der Schlitz 27 weist hierfür eine geringfügig kleinere lichte Weite verglichen mit dem Außendurchmesser des Silikonschlauches auf. Der Dosierkolben 20 wird in Anhäufung 25 aus Partikeln eingebettet, so dass der Dosierkolben 20 vollständig mit Partikeln umgeben ist. Durch entsprechendes Ziehen an dem in Fig. 4 nicht dargestellten Zugseil 21 wird nun der Dosierkolben 20 durch die durchgehende Öffnung 5 hindurch gezogen. Dabei werden die aus der Anhäufung 25 mitgenommenen Partikel durch den Dosierkolben 20 auf der inneren Oberfläche 7 der durchgehenden Öffnung 5 verteilt.

Nun wird der Silikonschlauch aus dem Schlitz 27 des Behälters 23 heraus genommen. Dann wird der Dosierkolben 20 nochmals durch die durchgehende Öffnung 5 gezogen, um die Verteilung der Partikel, die zuvor in die durchgehende Öffnung 5 eingebracht worden sind, über die gesamte innere Oberfläche der durchgehende Öffnung 5 zu verteilen.

Das oben beschriebene Verfahren zum Aufbringen der Partikel auf die innere Oberfläche der durchgehenden Öffnung 5 wird dann bei Bedarf für die weiteren durchgehenden Öffnungen des Schlauchs 2 wiederholt. Anschließend wird das Zugseil oder -band in die durchgehende Öffnung(en) des Außenlumens 5 eingeführt und die Herstellung der Führungseinrichtung ist damit beendet.

Um die medizinische Vorrichtung fertig zu stellen, wird anschließend die Führungseinrichtung mit den weiteren Elementen der medizinischen Vorrichtung verbunden.

### Bezugszeichenliste

- 1: Innenschlauch
- 2: Außenschlauch
- 5: durchgehende Öffnung als Außenlumen
- 6: Seil
- 7: innere Oberfläche des Außenlumens 5
- 8: äußere Oberfläche (Mantelfläche) des Führungsseils 6
- 9: Partikel
- 10: Doppelpfeil
- 12: Erweiterung der Öffnung 5 in dem Außenlumen durch Verschleiß
- 14: markierter Bereich
- 20: Dosierkolben
- 21: Führungsseil
- 23: Behälter
- 25: Anhäufung von zylinder- oder kugelförmigen Partikeln in dem Behälter 23
- 27: Schlitz
- 28: Seitenwand
- 100: medizinische Vorrichtung

## Patentansprüche

1. Führungseinrichtung mit einem Polymer- und/oder Kunststoffschlauch (2), insbesondere Schlauch enthaltend ein Polysiloxan, in dessen durchgehender Öffnung ein in der Öffnung bewegliches Seil (6) geführt ist, **dadurch gekennzeichnet, dass** auf der äußeren Oberfläche (8) des Seils (6) und/oder der inneren Oberfläche (7) der durchgehenden Öffnung (5) ein Belag angeordnet ist, der überwiegend aus im Wesentlichen kugel- oder zylinderförmigen und zumindest auf der jeweiligen Oberfläche frei beweglichen Partikeln (9) besteht.

2. Führungseinrichtung nach Anspruch 1, **dadurch gekennzeichnet, dass** die Partikel (9) des Belags nicht zerreibbar und/oder nicht degradierbar sind.

3. Führungseinrichtung nach einem der vorhergehenden Ansprüche, **dadurch gekennzeichnet, dass** die Partikel (9) einen Durchmessermittelwert größer oder gleich 20 µm, bevorzugt nicht größer als 60 µm, besonders bevorzugt 50 µm aufweisen.

4. Führungseinrichtung nach einem der vorhergehenden Ansprüche, **dadurch gekennzeichnet, dass** die Partikel (9) aus Korund, Quarzsand, Polyamid, Polyimid oder (Poly-)Tetrafluorethylen hergestellt sind.

5. Medizinische Vorrichtung, insbesondere Katheter oder implantierbare Elektrodenleitung, mit einer Führungseinrichtung nach einem der vorhergehenden Ansprüche.

6. Verfahren zur Herstellung einer Führungseinrichtung, insbesondere für einen Katheter, mit einem Kunststoff- und/oder Polymerschlauch (2), dessen durchgehende Öffnung (5) zur Führung eines in der Öffnung (2) beweglichen Seils (6) dient, **dadurch gekennzeichnet, dass** ein Dosierkolben (20) zuerst durch eine Anhäufung (25) von Partikeln (9), welche im Wesentlichen kugel- oder zylinderförmig und voneinander unabhängig beweglich sind, und anschließend durch die durchgehende Öffnung (5) des zuvor bereitgestellten Kunststoff- und/oder Polymerschlauchs (2) bewegt wird.

7. Verfahren nach Anspruch 6, **dadurch gekennzeichnet, dass** der Dosierkolben (20) anschließend noch mindestens ein weiteres Mal durch die durchgehende Öffnung bewegt wird.

8. Verfahren nach einem der Ansprüche 6 oder 7, **dadurch gekennzeichnet, dass** abschließend das Seil (6) in die Öffnung (5) eingeführt wird.

9. Verfahren zur Herstellung einer medizinischen Vorrichtung, insbesondere eines Katheters, **dadurch gekennzeichnet, dass** zunächst die Führungseinrichtung nach einem der Ansprüche 6 bis 8 hergestellt und anschließend die weiteren Elemente der medizinischen Vorrichtung an der Führungseinrichtung angeordnet werden.

## Claims

1. A slitting tool for slitting open a catheter shaft, comprising a housing, a blade (5) fastened to the housing, a push button (1), which can be actuated from the outside on the housing, and clamping forceps (2), which are fixedly mounted in the housing, the clamping forceps (2) being partially located inside the housing and composed of two clamping legs, which are connected to each other at the respective bases thereof located in the housing and have a mutual pretension, and which cause the fixation of an electrode lead (11) in the idle state on the peripheries thereof located outside the housing, a spreading element (8) being further displaceably mounted in the housing, and the push button (1), the spreading element (8), and the clamping forceps (2) cooperating with each other in such a way that an actuation of the push button (1) cancels the idle state of the clamping forceps (2) and causes the clamping forceps (2) and the spreading element (8) to be displaced with respect to each other and the fixation in the periphery of the clamping forceps (2) to be opened by moving the two legs away from each other by way of the spreading element (8), **characterized in that** the push button (1) is connected to the spreading element (8) so that the actuation of the push button (1) causes a displacement of the spreading element (8) in the direction of the base of the clamping forces (2) which is fixedly mounted in the housing, so that the two legs are pushed apart from each other.

2. The slitting tool according to claim 1, **characterized in that** the push button (1) has a first position protruding from the housing in the idle state and a second position located in the housing after actuation.

3. The slitting tool according to claim 2, **characterized in that** one or more reset elements cause a return movement of the push button (1) from the second position into the first position.

4. The slitting tool according to claim 3, **characterized in that** the reset element is or the reset elements are springs (9), which act between the housing and the push button (1).

5. A slitting tool according to any one of claims 1 to 4, **characterized in that** spreading element (8) and the base of the clamping forceps (2) are displaced toward each other upon actuation of the push button (1).

6. A slitting tool according to any one of claims 1 to 5, **characterized in that** the spreading element (8) is positioned between the two legs of the clamping forceps (2), or assumes the position between the two legs upon actuation of the push button (1).

7. The slitting tool according to claim 1, **characterized in that** the periphery of the clamping forceps (2) is formed by the open ends of the clamping forceps (2), which are each composed of a round or prism-shaped half shell for accommodation of the electrode lead (11), whereby the fixation of the electrode lead (11) is caused while the catheter is being slit open.

8. The slitting tool according to claim 7, **characterized in that** the half shells of the clamping forceps (2) form an enclosed cylindrical space in the idle state, which has a longitudinal axis that is situated so that the electrode lead (11) located in this space and, thus the catheter, are positioned relative to the blade (5) so that the blade (5) can slit open the catheter in the longitudinal direction thereof.

9. The slitting tool according to claim 8, **characterized in that** the blade (5) has a guide which extends parallel to the longitudinal axis.

## Revendications

1. Outil de rainurage destiné à fendre une tige de cathéter, comprenant un boîtier, une lame (5) fixée au boîtier, un bouton poussoir (1) actionnable de l'extérieur sur le boîtier, ainsi qu'une pince de serrage (2) agencée fixement dans le boîtier, dans lequel la pince de serrage (2) se trouve partiellement à l'intérieur du boîtier, tout en étant constituée de deux jambes de serrage reliées entre elles par leur base située dans le boîtier et exerçant une précontrainte l'une sur l'autre, et provoquant la fixation d'une ligne d'électrode (11), à l'état de repos, sur leur périphérie située à l'extérieur du boîtier, et dans lequel un élément d'espacement (8) est en outre monté de façon coulissante dans le boîtier, et dans lequel le bouton poussoir (1), l'élément d'espacement (8) et la pince de serrage (2) coopèrent de manière à ce qu'un actionnement du bouton poussoir (1) sort la pince de serrage (2) de l'état de repos, provoque un déplacement mutuel de la pince de serrage (2) et de l'élément d'espacement (8) ainsi qu'une ouverture de la fixation à la périphérie de la pince de serrage (2), en éloignant les deux jambes l'une à distance de l'autre à l'aide de l'élément d'espacement (8), **caractérisé en ce que** le bouton poussoir (1) est relié à l'élément d'espacement (8), de sorte que l'actionnement du bouton poussoir (1) provoque un déplacement de l'élément d'espacement (8) dans la direction de la base de la pince de serrage (2) agencée fixement dans le boîtier, de manière à écarter les deux jambes.

2. Outil de rainurage selon la revendication 1, **caractérisé en ce qu'**à l'état de repos, le bouton poussoir (1) présente une première position faisant saillie hors du boîtier et une deuxième position située dans le boîtier après l'actionnement.

3. Outil de rainurage selon la revendication 2, **caractérisé en ce qu'**un ou plusieurs éléments de rappel provoquent un mouvement retour du bouton poussoir (1), de la deuxième vers la première position.

4. Outil de rainurage selon la revendication 3, **caractérisé en ce que** l'élément de rappel ou les éléments de rappel sont des ressorts (9) agissant entre le boîtier et le bouton poussoir (1).

5. Outil de rainurage selon l'une des revendications 1 à 4, **caractérisé en ce que** l'élément d'espacement (8) et la base de la pince de serrage (2) se déplacent l'un vers l'autre lors de l'actionnement du bouton poussoir (1).

6. Outil de rainurage selon l'une des revendications 1 à 5, **caractérisé en ce que** l'élément d'espacement (8) est positionné entre les deux jambes de la pince de serrage (2), ou se met dans la position entre les deux jambes lors de l'actionnement du bouton poussoir (1).

7. Outil de rainurage selon la revendication 1, **caractérisé en ce que** la périphérie de la pince de serrage (2) est formée par les extrémités ouvertes de la pince de serrage (2), lesquelles sont respectivement constituées d'une demi-coque ronde ou prismatique, pour la réception de la ligne d'électrode (11), provoquant ainsi la fixation de la ligne d'électrode (11) pendant le rainurage du cathéter.

8. Outil de rainurage selon la revendication 7, **caractérisé en ce qu'**à l'état de repos, les demi-coques de la pince de serrage (2) forment un espace cylindrique fermé, lequel présente un axe longitudinal agencé de manière à ce que la ligne d'électrode (11) située dans cet espace et donc le cathéter soient agencés de telle façon par rapport à la lame (5), que la lame (5) peut fendre le cathéter dans son sens longitudinal.

9. Outil de rainurage selon la revendication 8, **caractérisé en ce que** la lame (5) comporte un guidage s'étendant parallèlement à l'axe longitudinal.
